# EUROPEAN PATENT SPECIFICATION

(11) **EP 3 108 845 B1**
(45) Date of publication and mention of the grant of the patent: **19.12.2018**
(21) Application number: 15752247.5
(22) Date of filing: 18.02.2015
(51) Int. Cl.: A61B 34/30, A61B 34/37, A61B 90/00, A61B 17/28, A61B 34/00

(54) **SURGICAL MANIPULATOR MANIPULATING DEVICE AND SURGICAL MANIPULATOR SYSTEM**
BEDIENUNGSVORRICHTUNG FÜR CHIRURGISCHEN MANIPULATOR UND CHIRURGISCHES MANIPULATORSYSTEM
DISPOSITIF DE COMMANDE DE MANIPULATEUR CHIRURGICAL ET SYSTÈME DE MANIPULATEUR CHIRURGICAL

(30) Priority: 21.02.2014 JP 2014031651
(43) Date of publication of application: 28.12.2016
(73) Proprietor: Olympus Corporation, Hachioji-shi, Tokyo 192-8507 (JP)
(72) Inventor: OGAWA, Ryohei, Hachioji-shi, Tokyo 192-8507 (JP); YANAGIHARA, Masaru, Hachioji-shi, Tokyo 192-8507 (JP); KISHI, Kosuke, Hachioji-shi, Tokyo 192-8507 (JP)
(74) Representative: Schicker, Silvia
(86) International application number: PCT/JP2015/054356
(87) International publication number: WO 2015/125797

(56) References cited:
- EP-A1- 1 982 637
- EP-A1- 3 025 629
- EP-A1- 3 025 633
- EP-A2- 1 782 744
- WO-A1-2015/012163
- JP-A- 2002 224 016
- JP-A- 2009 101 077
- US-A1- 2003 018 237

## Description

### {Technical Field}

The present invention relates to a surgical-manipulator manipulating device and a surgical manipulator system.

### {Background Art}

In the related art, in surgical manipulator systems employing a master-slave system, there is a known surgical manipulator system in which a large movement that controls the degree of freedom at the basal end of a slave device is electrically driven, a small movement that controls the degree of freedom at the distal end of the slave device is manually performed, and thus, an operator manipulates the slave device while feeling a force sensation (for example, see Patent Literature 1). As a further example, Patent Literature 2 discloses an endoscope system including an endoscope, a manipulating unit, and controlling unit. The endoscope has an elongated and flexible insertion tube to be used in combination with a therapeutic instrument and having a distal section accommodating therein at least an optical system for imaging and presenting a longitudinal direction. The controlling unit controls the therapeutic instrument based on a command from the manipulating unit.Still further, Patent Literature 3 discloses an endoscopic operation assisting device including first and second handle members, which are openable and closable, and an operation switch. In particular, the first handle member includes, in an opening and closing surface portion thereof, a groove forming an insertion section holding hole for holding an insertion section of an endoscope, and a step portion on a surface portion contraposed to the opening and closing surface portion. On the other hand, the second handle member includes, in an opening and closing surface portion, a groove forming the insertion section holding hole.

### {Citation List}

### {Patent Literature}

{Patent Literature 1} Publication of Japanese Patent No. 5323578
{Patent Literature 2} European Patent Application Publication No. 1 782 744 A2
{Patent Literature 3} European Patent Application Publication No. 1 982 637 A1

### {Summary of Invention}

### {Technical Problem}

However, in the case in which the slave device is a treatment tool for treating an affected portion by being placed so as to protrude from the distal end of a flexible endoscope, it is difficult to employ the surgical manipulator system of Patent Literature 1.

In other words, in general, forward/backward movements until the distal end of the flexible endoscope reaches a treatment target site and bending manipulations of the distal end are manually performed. Manual manipulation is suitable because the procedure needs to be performed while feeling the reaction or the like from the organ in order to insert the flexible endoscope so as to conform to a bent organ.

Also, if the endoscope is positioned by electrically driving the endoscope, as with the prior art, when performing treatment by using a treatment tool after manually adjusting the distal-end position of the flexible endoscope, there is a problem in that the operational feeling is lost because there is a large difference as compared with the manual manipulation of the flexible endoscope that has previously been performed, thus making manipulation thereof difficult. Furthermore, when moving the distal end of the treatment tool, because the treatment tool is more likely to come into contact with a peripheral organ when moving the position thereof by a large distance from one affected portion to another affected portion as compared with when moving the position thereof by a small distance in the area around one affected portion, the large-distance movement needs to be performed while feeling the reaction from the peripheral organ.

In contrast, with regard to small movements that affect the degree of freedom at the treatment-tool distal end, because such movements are used to make fine incisions in target tissue or to perform removal thereof, finer movements are required. Accordingly, there is a problem in that, when the degree of freedom at the distal end of the slave device is manually manipulated, as with the prior art, the manipulation ends up being rough.

The present invention has been conceived in light of the above-described circumstances, and an object thereof is to provide a surgical-manipulator manipulating device and a surgical manipulator system with which, when electrically driving a treatment tool that is used together with a flexible endoscope, it is possible to manipulate the treatment tool without losing the operational feeling.

### {Solution to Problem}

In order to achieve the above-described object, the present invention provides the following solutions.

The invention is defined by appended independent claim 1. Preferred embodiments are given in the dependent claims. An aspect of the present invention is a surgical-manipulator manipulating device for manipulating a surgical manipulator including the features of claim 1. A surgical-manipulator manipulating device may include: a treatment-tool distal-end portion having at least one electrically manipulated joint disposed at a distal end of an inserted portion of a flexible endoscope and a manual moving mechanism that moves the position of the treatment-tool distal-end portion, the surgical-manipulator manipulating device including: a manual manipulating portion that manipulates the moving mechanism; and an electric manipulating portion that manipulates the joint of the treatment-tool distal-end portion; wherein the manual manipulating portion includes a movable portion that moves the treatment-tool distal-end portion in accordance with the amount of displacement, and the electric manipulating portion includes a manipulation input portion that drives the joint, and can be secured to the movable portion.

With the surgical-manipulator manipulating device according to this aspect, via the operation of the moving mechanism performed by displacing the movable portion of the manual manipulating portion, the treatment-tool distal-end portion disposed at the distal end of the inserted portion of the flexible endoscope inserted into the body is manually
moved by an amount in accordance with the amount by which the movable portion is displaced. Because a large movement of the treatment-tool distal-end portion is likely to cause interference with a peripheral organ, by manually performing such a movement, it is possible to smoothly move the treatment-tool distal-end portion while feeling the reaction from the organ. Then, after the treatment-tool distal-end portion is positioned at the affected portion, the operator manipulates the manipulation input portion of the electric manipulating portion, and thus, the joint of the treatment-tool distal-end portion is electrically driven in accordance with the input made via the manipulation input portion. By doing so, detailed procedures performed on the affected portion can electrically be performed in a precise manner.

In this case, because the manipulation for a large movement of the treatment-tool distal-end portion, which is performed after the manual manipulation for the insertion of the flexible endoscope, is also manually performed, the operational feeling of the operator is not lost, making it possible to achieve easy manipulation. In addition, because the electric manipulating portion is provided so that the electric manipulating portion can be secured to the movable portion, when the operator moves the electric manipulating portion, it is possible to move the movable portion to which the electric manipulating portion is secured. In other words, it is possible for the operator to perform both the manual manipulation for manually performing a large movement of the treatment-tool distal-end portion and the electric manipulation for electrically moving the joint of the treatment-tool distal-end portion in a precise manner in a state in which the electric manipulating portion or the movable portion continues to be gripped, and it is possible to efficiently perform these procedures without having to release the hand.

In the above-described aspect, the surgical manipulator is provided with a flexible portion in which the treatment-tool distal-end portion is secured to a distal end thereof, and the movable portion is secured to the flexible portion so as to be able to move the flexible portion in a longitudinal direction thereof.

By doing so, when the operator grips the electric manipulating portion and moves the electric manipulating portion itself, the movable portion secured to the electric manipulating portion is moved. In the case in which the movable portion is secured to the flexible portion, by moving the electric manipulating portion in the longitudinal direction of the flexible portion, it is possible to manually move the treatment-tool distal-end portion, which is attached
to the flexible portion and the distal end of the flexible portion via the movable portion secured to the electric manipulating portion, in the longitudinal direction of the flexible portion.

In the above-described aspect, the moving mechanism is provided with a bending portion that pivots the treatment-tool distal-end portion, the manual manipulating portion may be provided with a base portion, and the movable portion may be attached to the base portion in a pivotable manner, and may be connected to the bending portion via a wire.

By doing so, when the movable portion is pivoted with respect to the base portion, this pivoting movement is transmitted to the bending portion via the motive-power transmitting member, and, due to the operation of the bending portion, the treatment-tool distal-end portion is pivoted, and the position thereof is moved. By doing so, by moving the movable portion so as to be pivoted while the operator keeps gripping the electric manipulating portion or the movable portion, it is possible to move the position of the treatment-tool distal-end portion by manually pivoting the treatment-tool distal-end portion disposed at the distal end of the flexible endoscope.

In the above-described aspect, the manual manipulating portion is provided with a pivoting portion that is attached to the base portion in a pivotable manner. The movable portion may be formed in a cylindrical shape through which the flexible portion passes and is supported by the pivoting portion so as to be movable along a longitudinal direction thereof.

By doing so, by pivoting the movable portion, which passes through the flexible portion, together with the pivoting portion secured to the flexible portion, the bending portion is bent via the motive-power transmitting member, and thus, the position can be moved by means of pivoting of the treatment-tool distal-end portion. In addition, by moving the secured portion secured to the flexible portion with respect to the pivoting portion in the longitudinal direction of the flexible portion, it is possible to move the treatment-tool distal-end portion forward/backward in the longitudinal direction of the flexible portion.

The above-described aspect may be provided with a movement restricting mechanism that restricts a movement range of the movable portion in a longitudinal direction with respect to the pivoting portion.

By doing so, the movement of the movable portion in the longitudinal direction is restricted by the movement
restricting mechanism, and it is possible to restrict manually performed forward/backward movements of the treatment-tool distal-end portion so that they do not become excessively large.

The above-described aspect may be provided with a locking mechanism that locks the pivoting portion at an arbitrary pivoting position with respect to the base portion.

By doing so, via the operation of the locking mechanism, it is possible to hold the position of the treatment-tool distal-end portion, which is positioned with respect to the affected portion by manipulating the manual manipulating portion, so as to be immobilized. Also, by manipulating the electric manipulating portion in this state, it is possible to electrically operate the joint of the treatment-tool distal-end portion, and thus, it is possible to precisely treat the affected portion.

In the above-described aspect, the electric manipulating portion may be secured to the movable portion in an attachable/detachable manner.

By doing so, by removing the electric manipulating portion from the movable portion, it is possible to manipulate the electric manipulating portion separated from the manual manipulating portion.

The above-described aspect may be provided with a manipulation table to which the base portion is attached, wherein the surgical manipulator may be provided with a motor unit that drives the joint at a basal end of the flexible portion, and the motor unit may be attached to the manipulation table in a pivotable manner.

By doing so, when the flexible portion is displaced by moving the movable portion with respect to the base portion attached to the manipulation table, an external force applied to the movable portion is transmitted to the motor unit attached to the basal end of the flexible portion via the flexible portion; however, the motor unit is pivoted with respect to the manipulation table, and thus, it is possible to prevent an excessive external force from being applied to a connecting portion between the flexible portion and the motor unit.

In the above-described aspect, the base portion may be attached to the manipulation table in an attachable/detachable manner.

By doing so, it is possible to easily exchange the base portion by removing the base portion from the manipulation table.

In the above-described aspect, the motive-power transmitting member is a wire, and the surgical-manipulator manipulating device may be provided with a guide tube that accommodates the wire in a state in which the wire passes therethrough and that is connected, at a distal end thereof, to the moving mechanism. A basal end of the guide tube is secured to the base portion (e.g., in an attachable/detachable manner), and one end of the wire is secured to the pivoting portion. The surgical-manipulator manipulating device may further comprise a columnar portion to which one end of the wire is secured and around which the wire is wound may be attached to the pivoting portion in an attachable/detachable manner.

By doing so, after completing treatment, by removing the guide tube from the base portion, and by removing the columnar portion from the pivoting portion, thus making the guide tube, the columnar portion, and the wire disposable, it is possible to easily exchange these components, and it is possible to reuse the base portion, the pivoting portion, and so forth.

Another aspect of the present invention is a surgical manipulator system provided with any one of the surgical-manipulator manipulating devices described above.

### {Advantageous Effects of Invention}

The present invention affords an advantage in that, when a treatment tool that is used together with a flexible endoscope is employed as a slave device, it is possible to
manipulate the treatment tool without losing the operational feeling.

### {Brief Description of Drawings}

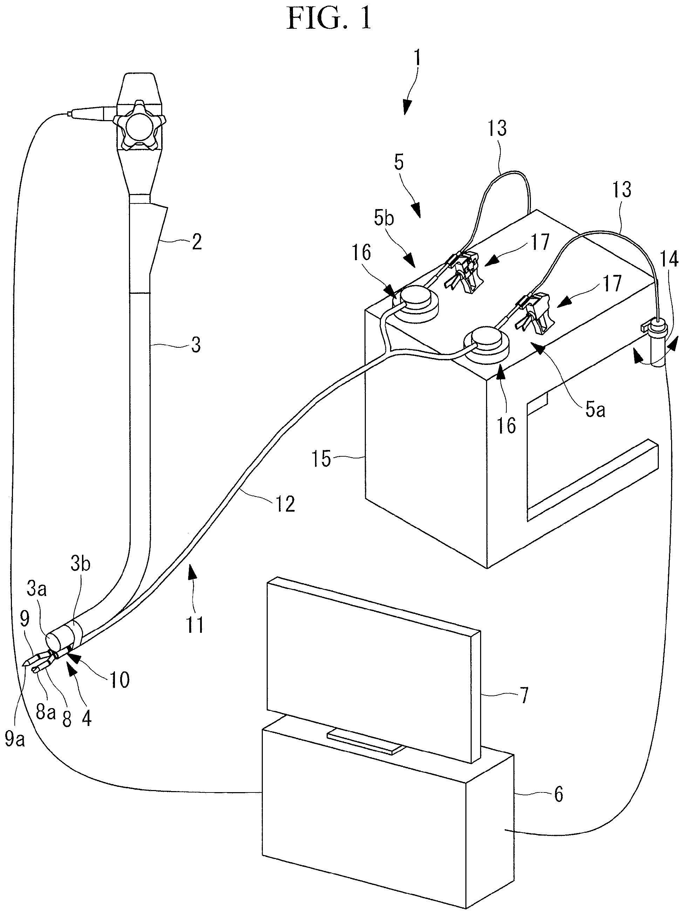

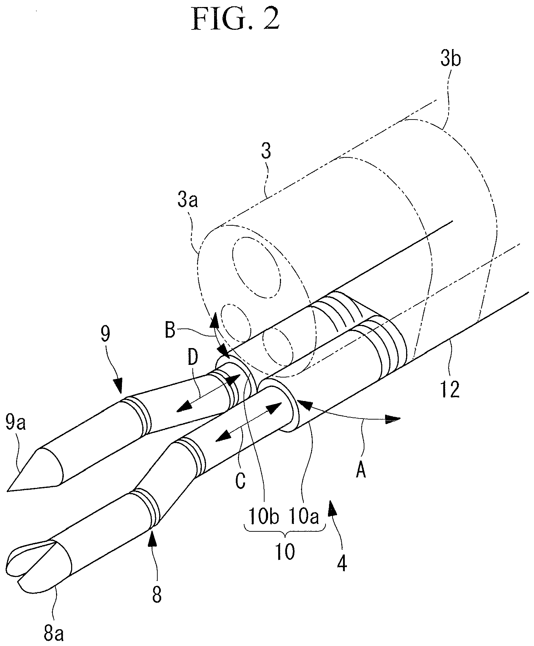

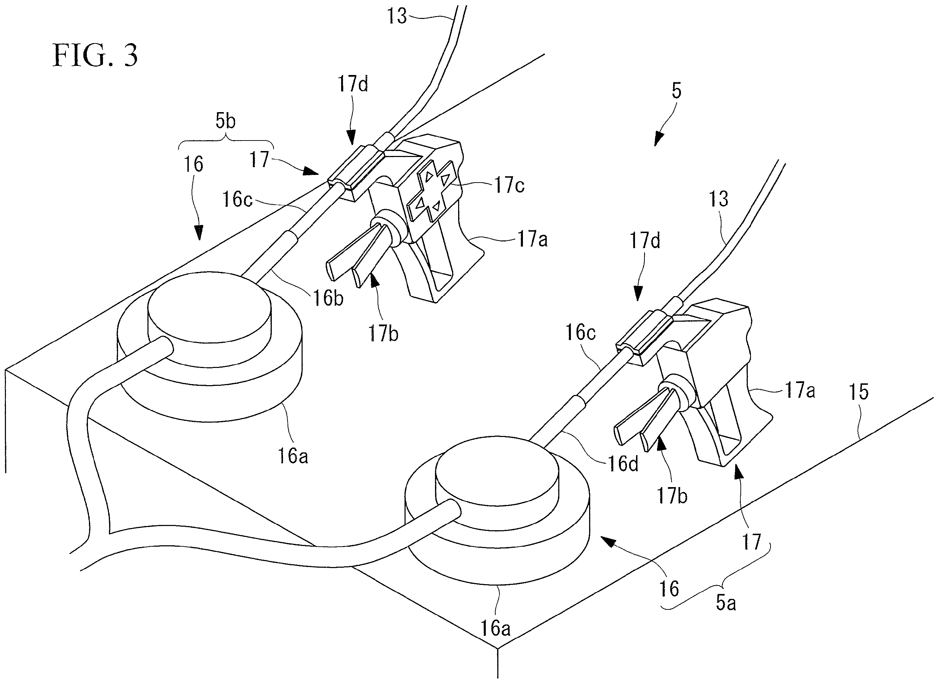

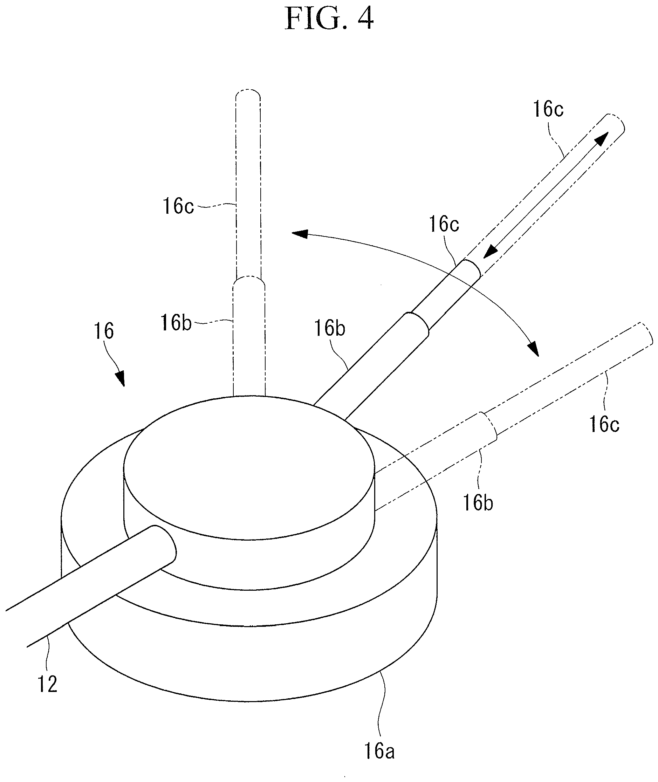

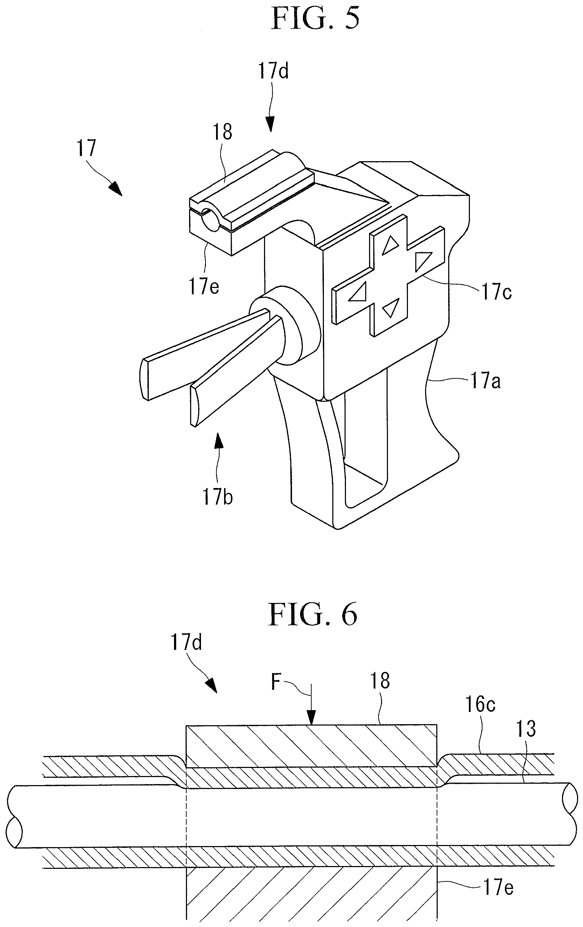

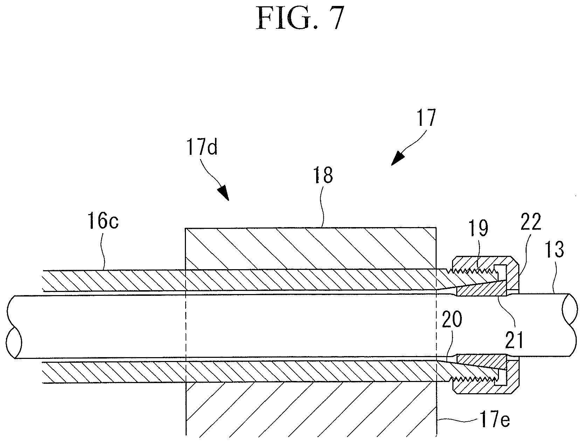

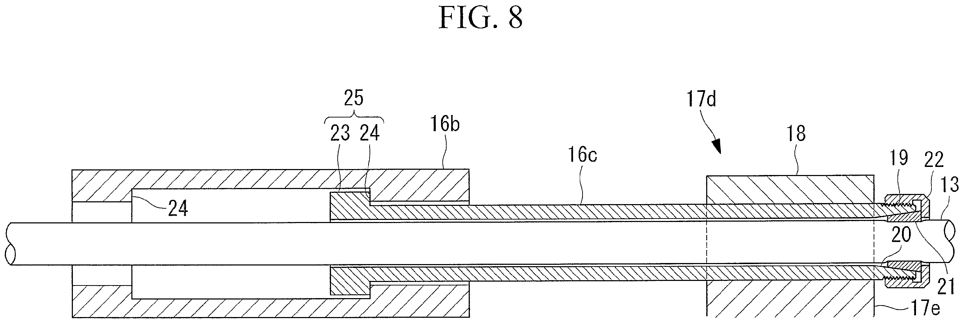

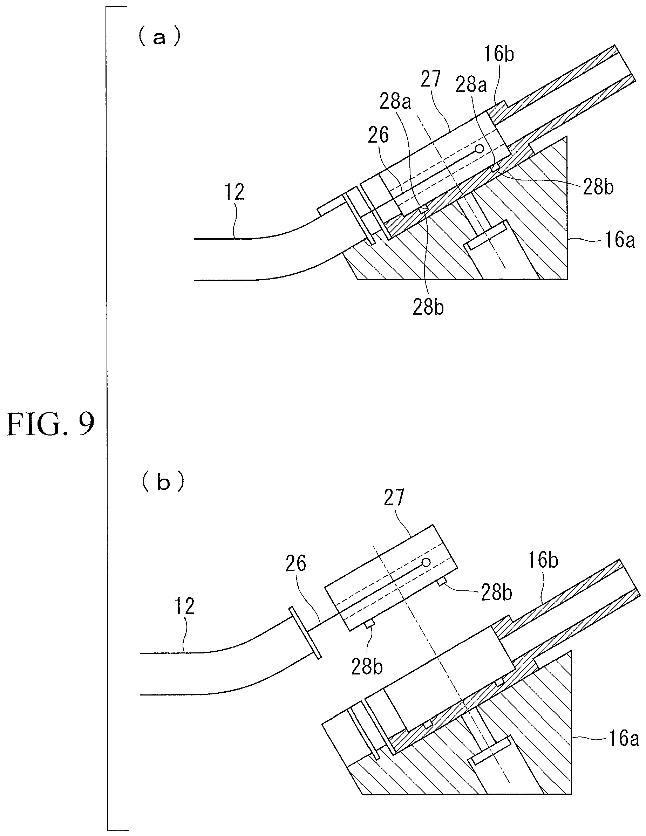

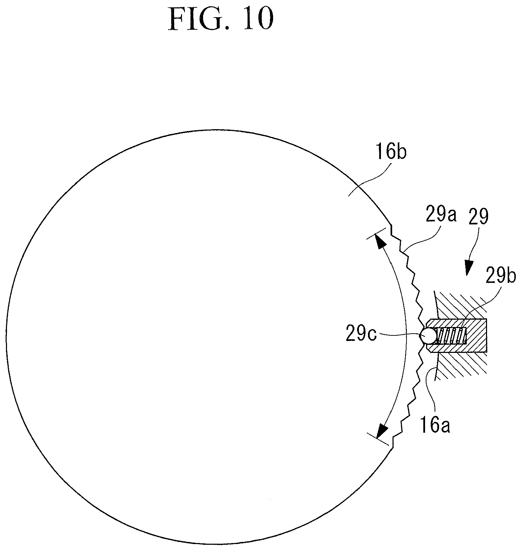

{Fig. 1} Fig. 1 is an overall configuration diagram showing a surgical manipulator system according to an embodiment of the present invention.
{Fig. 2} Fig. 2 is an enlarged perspective view showing a manipulator that is attached to the distal end of a flexible endoscope in the surgical manipulator system in Fig. 1.
{Fig. 3} Fig. 3 is a perspective view showing an example of a surgical-manipulator manipulating device according to an embodiment of the present invention, for the surgical manipulator system in Fig. 1.
{Fig. 4} Fig. 4 is a perspective view showing a manual manipulating portion of the surgical-manipulator manipulating device in Fig. 3.
{Fig. 5} Fig. 5 is a perspective view showing an electric manipulating portion of the surgical-manipulator manipulating device in Fig. 3.
{Fig. 6} Fig. 6 is a partial longitudinal cross-sectional view showing an example of a secured portion between a movable portion of the manual manipulating portion in Fig. 4 and the electric manipulating portion in Fig. 5.
{Fig. 7} Fig. 7 is a partial longitudinal cross-sectional view showing a modification of the secured portion in Fig. 6.
{Fig. 8} Fig. 8 is a partial longitudinal cross-sectional view showing a movement restricting mechanism provided in the manual manipulating portion in Fig. 4.
{Fig. 9} Fig. 9 is a longitudinal cross-sectional view showing, in a modification of the manual manipulating portion in Fig. 4, (a) a state in which a mechanism including a wire is attached to a pivoting portion, and (b) a state in which the mechanism including the wire is removed from the pivoting portion.
{Fig. 10} Fig. 10 is a partial longitudinal cross-sectional view for explaining an example of a locking mechanism of the pivoting portion of the manual manipulating portion in Fig. 4.

### {Description of Embodiment}

A surgical manipulator system 1 according to an embodiment of the present invention will be described below with reference to the drawings.

As shown in Fig. 1, the surgical manipulator system 1 according to this embodiment is provided with: a manipulator 4 that is secured to a distal-end portion of an inserted portion 3 of a flexible endoscope 2 by means of a securing tool 3b, such as a belt or the like, and that is operated in that state; a surgical-manipulator manipulating device 5 for manipulating the manipulator 4; a control portion 6 that controls the manipulator 4 on the basis of manipulation instructions input via the surgical-manipulator manipulating device 5; and a monitor 7 on which an image acquired by using the flexible endoscope 2 is displayed.

As shown in Fig. 2, the manipulator 4 is provided with: two treatment-tool distal-end portions 8 and 9 that are placed in the viewing-field area of the flexible endoscope 2 in a state in which the treatment-tool distal-end portions 8 and 9 are protruded forward from a distal-end surface 3a of the inserted portion 3; a moving mechanism 10 for moving the individual treatment-tool distal-end portions 8 and 9 as a whole; and a treatment-tool distal-end-portion driving mechanism 11 for electrically operating the individual treatment-tool distal-end portions 8 and 9. The treatment-tool distal-end portions 8 and 9 have one or more electrically manipulated joints, and are provided with gripping forceps 8a, energy forceps 9a, or the like (hereinafter, also referred to as "end effectors") at the distal ends thereof.

The moving mechanism 10 is provided with bending arms (manual bending portions) 10a and 10b that pivot the individual treatment-tool distal-end portions 8 and 9 in predetermined directions A and B. In addition, the moving mechanism 10 supports the treatment-tool distal-end portions 8 and 9 so as to be movable in directions C and D in which the treatment-tool distal-end portions 8 and 9 are moved forward/backward with respect to the bending arms 10a and 10b.

By doing so, the moving mechanism 10 is configured so that, by pivoting the bending arms 10a and 10b in the predetermined directions A and B, the overall directions of the treatment-tool distal-end portions 8 and 9 can be changed, so that the entire treatment-tool distal-end portions 8 and 9 can be moved forward/backward by moving the treatment-tool distal-end portions 8 and 9 in the directions C and D in which the treatment-tool distal-end portions 8 and 9 move forward/backward with respect to the bending arms 10a and 10b, and so that the end effectors 8a and 9a at the distal ends can be moved forward or backward in the viewing-field area of the flexible endoscope 2.

A long, thin, flexible guide tube 12 is connected to the basal ends of the bending arms 10a and 10b of the moving mechanism 10. A plurality of wires (not shown) pass therethrough along the entire length of the guide tube 12. Distal ends of the wires are connected to the bending arms 10a and 10b so as to pivot the bending arms 10a and 10b by means of tensile forces. Basal ends of the wires are connected to a surgical-manipulator manipulating device 5 (described later) that is disposed at the basal end of the guide tube 12.

Long, thin, flexible portions 13 having flexibility are individually connected to basal ends of the treatment-tool distal-end portions 8 and 9. A plurality of wires (not shown) for driving the joints of the individual treatment-tool distal-end portions 8 and 9 pass therethrough along the entire lengths of the individual flexible portions 13. Distal ends of the wires are connected to the individual joints so that the individual joints can independently be pivoted by means of tensile forces.

In addition, the flexible portions 13 are connected to motor units 14 at the basal ends thereof. The motor units 14 are provided with a plurality of motors (not shown) that separately apply tensile forces to the individual wires passing through the flexible portions 13, and are driven on the basis of instructions from the control portion 6. As shown in Fig. 1, each motor unit 14 is suspended at a side surface of a manipulation table 15 so as to be pivotable about a horizontal axis with the portion connecting with the flexible portion 13 facing vertically upward, and so as to allow position adjustment so that doctors can place the motor unit 14 at an optimal position.

The guide tube 12 is provided with channels (not shown) that pass therethrough over the entire length thereof in the longitudinal direction, and the flexible portions 13 are inserted into the channels so as to be movable in the longitudinal directions thereof. The forward/backward operation of the treatment-tool distal-end portions 8 and 9 with respect to the bending arms 10a and 10b is executed by manually moving the flexible portions 13 themselves in the longitudinal directions at the basal end of the guide tube 12.

As shown in Figs. 1 and 3, the surgical-manipulator manipulating device 5 according to this embodiment is provided with a pair of left and right manipulation devices 5a and 5b in order for an operator to perform manipulation by using both hands. For example, the left manipulation device 5a is for manipulating the left treatment-tool distal-end portion 8, and the right manipulation device 5b is for manipulating the right treatment-tool distal-end portion 9.

The individual manipulation devices 5a and 5b are provided with manual manipulating portions 16 and electric manipulating portions 17.

As shown in Fig. 4, the manual manipulating portions 16 are provided with: two base portions 16a that are secured to the manipulation table 15 in a manner that allows position adjustment or in an attachable/detachable manner so that each doctor can place the manual manipulating portions 16 at optimal positions; cylindrical pivoting portions 16b that are supported by the base portions 16a so as to be pivotable about axes extending in the top-to-bottom directions; and cylindrical movable portions 16c that are disposed inside the pivoting portions 16b and that are supported so as to be movable in the longitudinal directions thereof.

The movable portions 16c are permitted to move only in the longitudinal directions relative to the pivoting portions 16b, and are restricted in terms of relative movement in the radial direction. By doing so, when forces in the radial directions that are aligned with the pivoting directions of the pivoting portions 16b are applied to the movable portions 16c, the movable portions 16c are pivoted together with the pivoting portions 16b.

The flexible portions 13 extend so as to pass through the movable portions 16c, the pivoting portions 16b, and the base portions 16a, and are secured in an attachable/detachable manner to the movable portions 16c by means of a method described later.

As shown in Fig. 5, each electric manipulating portion 17 is provided with: a gripping portion 17a that is gripped by the operator; a first manipulation input portion 17b that is manipulated by being held between the thumb and the index finger of the hand (for example, right hand) holding the gripping portion 17a; a second manipulation input portion 17c, such as a four-way key, that is also manipulated by the thumb of the gripping hand; and an attaching/detaching portion 17d for securing the electric manipulating portion 17 to the movable portion 16c of the manual manipulating portion 16 in an attachable/detachable manner.

The first manipulation input portions 17b are configured so that instruction signals for, for example, opening/closing the gripping forceps 8a and energizing the energy forceps 9a at the distal ends of the treatment-tool distal-end portions 8 and 9 are input. In addition, the second manipulation input portions 17c are configured so that instruction signals for, for example, moving the end effectors 8a and 9a in the top-to-bottom directions or in the left-to-right directions by driving the joints of the treatment-tool distal-end portions 8 and 9 are input.

In addition, as shown in Fig. 6, each attaching/detaching portion 17d is provided with a holding member 18 that holds the movable portion 16c in the radial direction between a mounting portion 17e of the electric manipulating portion 17 and the holding member 18. When the pressure in the direction indicated by an arrow F is applied to the holding member 18 by fastening a screw or the like, the holding member 18 presses the movable portion 16c in the radial direction so that the electric manipulating portion 17 is secured to the movable portion 16c by means of friction, and also so that a portion of the pressed movable portion 16c is deformed in the radial direction.

By doing so, by pressing the inner surface of the movable portion 16c against the flexible portion 13 that passes through the interior of the movable portion 16c, the holding member 18 also secures the movable portion 16c and the flexible portion 13 by means of friction so as to prevent relative movement thereof.

Also, by securing the electric manipulating portion 17 to the flexible portion 13 via the movable portion 16c in this way, the manual manipulating portion 16 can be manipulated by using the electric manipulating portion 17 while preventing the electric manipulating portion 17, which the operator touches, from coming into direct contact with the flexible portion 13 in which the distal-end portion thereof is introduced inside the body of a patient.

The operation of the thus-configured surgical-manipulator manipulating device 5 and surgical manipulator system 1 according to this embodiment will be described below.

In order to treat an affected portion in the body of the patient by using the surgical manipulator system 1 according to this embodiment, first, the inserted portion 3 of the flexible endoscope 2, in which the two treatment-tool distal-end portions 8 and 9 are immobilized in a state in which the treatment-tool distal-end portions 8 and 9 are protruded from the distal-end surface 3a, is manually introduced into the body, and the position of the distal-end portion of the flexible endoscope 2 is adjusted so that the affected portion is placed in the viewing-field area of the flexible endoscope 2 while displaying an image captured by using the flexible endoscope 2 on the monitor 7 and checking the image.

Next, the operator brings the distal ends of the treatment-tool distal-end portions 8 and 9 closer to the affected portion while viewing the endoscope image of the area around the affected portion displayed on the monitor 7. In this case, first, the operator grips the gripping portions 17a of the two electric manipulating portions 17 with both hands and moves the electric manipulating portions 17 without manipulating the manipulation input portions 17b and 17c.

Specifically, by moving the electric manipulating portions 17 left and right and by pivoting the pivoting portions 16b with respect to the base portions 16a of the manual manipulating portions 16, the bending arms 10a and 10b of the moving mechanism 10 secured to the distal end of the inserted portion 3 are pivoted in the corresponding directions A and B, and the treatment-tool distal-end portions 8 and 9 are moved from left to right as a whole. In addition, by moving the movable portions 16c forward and backward with respect to the pivoting portions 16b of the manual manipulating portions 16 by moving the electric manipulating portions 17 back and forth, the treatment-tool distal-end portions 8 and 9 are moved, as a whole, forward and backward in the directions C and D.

By doing so, because the distal ends of the treatment-tool distal-end portions 8 and 9 are roughly positioned with respect to the affected portion, the operator manipulates the second manipulation input portions 17c provided in the electric manipulating portions 17 in this state to electrically drive the joints of the individual treatment-tool distal-end portions 8 and 9, thus achieving precise positioning thereof with respect to the affected portion, and then, the end effectors 8a and 9a are operated by manipulating the first manipulation input portions 17b. By doing so, it is possible to treat the affected portion.

In this case, with the surgical-manipulator manipulating device 5 according to this embodiment, because the procedure for roughly positioning the treatment-tool distal-end portions 8 and 9, which is performed subsequent to the manually performed manipulation for the insertion of the inserted portion 3 of the flexible endoscope 2, is also performed by manually manipulating the manual manipulating portions 16, there is an advantage in that the procedure can smoothly be performed without losing the operational feeling. Because the movements of the treatment-tool distal-end portions 8 and 9 in this case are relatively large movements in which the entire treatment-tool distal-end portions 8 and 9 are moved, the treatment-tool distal-end portions 8 and 9 come into contact with the peripheral organ in some cases; however, because the manual manipulation is performed while feeling the reaction from the organ, it is possible to prevent the organ from being excessively pressed by the treatment-tool distal-end portions 8 and 9.

In addition, because treatment, which is performed by using the treatment-tool distal-end portions 8 and 9 after the procedure for roughly positioning the treatment-tool distal-end portions 8 and 9, is electrically performed in accordance with the manipulation of the electric manipulating portions 17, there is an advantage in that it is possible to perform precise treatment by precisely operating the joints of the treatment-tool distal-end portions 8 and 9.

Furthermore, with this embodiment, because the electric manipulating portions 17 are secured to the movable portions 16c of the manual manipulating portions 16, to which the flexible portions 13 connected to the basal ends of the treatment-tool distal-end portions 8 and 9 are secured, it is possible to manually manipulate the moving mechanism 10 by moving the movable portions 16c of the manual manipulating portions 16 while the gripping portions 17a of the electric manipulating portions 17 are kept gripped. In other words, there is an advantage in that it is possible to continuously perform the manual manipulation for performing rough positioning and the electric manipulation for performing precise treatment without having to release the hands gripping the gripping portions 17a.

Note that, in this embodiment, by using the holding members 18, the electric manipulating portions 17 and the movable portions 16c are secured, and the movable portions 16c and the flexible portions 13 are also secured in the attaching/detaching portions 17d; alternatively, however, the holding members 18 may secure only the electric manipulating portions 17 and the movable portions 16c, as shown in Fig. 7. In this case, for example, the movable portions 16c and the flexible portions 13 may be secured by providing male screws 19 at outer surfaces of the movable portions 16c, by providing tapered inner surfaces 20 at inner surfaces thereof, and by pushing in tapered cylindrical bushes 21 placed between the tapered inner surfaces 20 and flexible portions 13 by using nuts 22 fastened to the male screws 19.

In addition, as shown in Fig. 8, movement restricting mechanisms 25 that restrict movement ranges of the movable portions 16c with respect to the pivoting portions 16b may be formed by providing protrusions 23 that protrude outward in the radial direction at the distal ends of the movable portions 16c and by providing stoppers 24 against which the protrusions 23 are abutted in the interior of the pivoting portions 16b. By doing so, it is possible to restrict excessive movement when moving the treatment-tool distal-end portions 8 and 9 forward/backward.

In addition, as shown in Figs. 9(a) and (b), in the manual manipulating portions 16, the basal ends of the guide tube 12 may be secured to the base portions 16a in an attachable/detachable manner, and columnar portions 27, to which one end of each wire 26 is secured, may be attached, in an attachable/detachable manner, to the pivoting portions 16b that are attached to the base portions 16a in a pivotable manner.

As shown in Fig. 9(a), because the columnar portions 27 secured to the pivoting portions 16b are also rotated, when the pivoting portions 16b are pivoted with respect to the base portions 16a while being attached to the guide tube 12 and the columnar portions 27, it is possible to pivot the bending arms 10a and 10b by winding the wires 26 accommodated in the guide tube 12 around the columnar portions 27 or by letting the wires 26 out. The columnar portions 27 can be secured to the pivoting portions 16b in a simple manner, for example, by fitting protrusions 28b of the columnar portions 27 to depressed portions 28a provided in the pivoting portions 16b.

In addition, after completing treatment, as shown in Fig. 9(b), by removing the guide tube 12 from the base portions 16a, and by removing the columnar portions 27 from the pivoting portions 16b, thus making the guide tube 12, the columnar portions 27, and the wires 26 disposable, it is possible to easily exchange these components, and it is possible to reuse the base portions 16a, the pivoting portions 16b, and so forth.

In addition, as shown in Fig. 10, locking mechanisms 29 that lock pivoting movements of the pivoting portions 16b with respect to the base portions 16a at arbitrary pivoting positions may be provided between the base portions 16a and the pivoting portions 16b. As the locking mechanisms 29, those including a plurality of depressed portions 29a provided on outer circumferential surfaces of the pivoting portions 16b in the circumferential direction and balls 29c that are biased, by using springs 29b, inward in the radial direction with respect to the depressed portions 29a may be employed.

By doing so, because the balls 29c are moved by the springs 29b inward in the radial direction of the pivoting portions 16b at positions aligned with the individual depressed portions 29a, it is possible to stop pivoting of the pivoting portions 16b by means of engagement between the depressed portions 29a and the balls 29c.

In addition, in this embodiment, although the gripping portions 17a are provided in the electric manipulating portions 17, there is no limitation thereto, and the gripping portions may be provided in the movable portions 16c.

In addition, treatment-tool identification information may be read at the base portions 16a. In this case, reading portions that read the treatment-tool identification information are provided in the pivoting portions 16b of the base portions 16a, and an identification-information retaining portion (for example, barcode, RFID, or the like) that retains the identification information is attached to a predetermined position of the treatment tool. For example, the treatment tool may be configured so as to be restricted in an initial position due to the mechanism thereof and the position that faces the reading potion when at the initial position may be employed as the predetermined position. Then, electric driving of the treatment tool may be started after completing reading.

### {Reference Signs List}

1 surgical manipulator system
2 flexible endoscope
3 inserted portion
4 manipulator (surgical manipulator)
5 surgical-manipulator manipulating device
8, 9 treatment-tool distal-end portion
10 moving mechanism
10a, 10b bending arm (bending portion)
13 flexible portion
14 motor unit
15 manipulation table
16 manual manipulating portion
16a base portion
16b pivoting portion
16c movable portion
17 electric manipulating portion
17a gripping portion
17b, 17c manipulation input portion
25 movement restricting mechanism
26 wire (motive-power transmitting member)
29 locking mechanism

## Claims

1. A surgical-manipulator manipulating device (5) for manipulating a surgical manipulator (4) including a treatment-tool distal-end portion (8, 9) having at least one electrically manipulated joint and a manual moving mechanism (10) that moves the position of the treatment-tool distal-end portion (8, 9), the surgical-manipulator manipulating device (5) comprising:
a manual manipulating portion (16) that manipulates the moving mechanism (10); and
an electric manipulating portion (17) that manipulates the joint of the treatment-tool distal-end portion (8, 9);
wherein:
the manual manipulating portion (16) includes a movable portion (16c) that moves the treatment-tool distal-end portion (8, 9) in accordance with an amount of displacement,
the electric manipulating portion (17) includes a manipulation input portion (17b, 17c) that drives the joint, and can be secured to the movable portion (16c), wherein
the surgical manipulator (4) includes a flexible portion (13) in which the treatment-tool distal-end portion (8, 9) is secured to a distal end thereof,
the movable portion (16c) is secured to the flexible portion (13) so as to be able to move the flexible portion (13) in a longitudinal direction thereof,
the moving mechanism (10) includes a bending portion (10a, 10b) that pivots the treatment-tool distal-end portion (8, 9),
the manual manipulating portion (16) includes a base portion (16a) and a pivoting portion (16b) that is attached to the base portion (16a) in a pivotable manner;
the movable portion (16c) is attached to the base portion (16a) in a pivotable manner, is connected to the bending portion (10a, 10b) via a wire (26) and is supported by the pivoting portion (16b) so as to be movable along a longitudinal direction thereof,
the surgical-manipulator manipulating device (5) further comprises a guide tube (12) that accommodates the wire (26) in a state in which the wire (26) passes therethrough and that is connected, at a distal end thereof, to the moving mechanism (10) including the bending portion (10a, 10b), and
a basal end of the guide tube (12) is secured to the base portion (16), and one end of the wire (26) is secured to the pivoting portion (16b).

2. A surgical-manipulator manipulating device (5) according to Claim 1, wherein the movable portion (16c) is formed in a cylindrical shape through which the flexible portion (13) passes.

3. A surgical-manipulator manipulating device (5) according to Claim 1 or 2, further comprising:
a movement restricting mechanism (25) that restricts a movement range of the movable portion (16c) in a longitudinal direction with respect to the pivoting portion (16b).

4. A surgical-manipulator manipulating device (5) according to any one of Claims 1 to 3, further comprising:
a locking mechanism (29) that locks the pivoting portion (16b) at an arbitrary pivoting position with respect to the base portion (16a).

5. A surgical-manipulator manipulating device (5) according to any one of Claims 1 to 4, wherein the electric manipulating portion (17) is secured to the movable portion (16c) in an attachable/detachable manner.

6. A surgical-manipulator manipulating device (5) according to any one of Claims 1 to 5, further comprising:
a manipulation table (15) to which the base portion (16a) is attached,
wherein the surgical manipulator (4) includes a motor unit (14) that drives the joint at a basal end of the flexible portion (13), and
the motor unit (14) is attached to the manipulation table (15) in a pivotable manner.

7. A surgical-manipulator manipulating device (5) according to Claim 6, wherein the base portion (16a) is attached to the manipulation table (15) in an attachable/detachable manner.

8. A surgical-manipulator manipulating device (5) according to Claim 1 or 2,
wherein the basal end of the guide tube (12) is secured to the base portion (16a) in an attachable/detachable manner, and
the surgical-manipulator manipulating device (5) further comprises a columnar portion (27) to which the one end of the wire (26) is secured and around which the wire (26) is wound, wherein the columnar portion is attached to the pivoting portion (16b) in an attachable/detachable manner.

9. A surgical manipulator system (1) comprising:
a surgical-manipulator manipulating device (5) according to any one of Claims 1 to 8.

## Patentansprüche

1. Vorrichtung (5) zum Manipulieren eines chirurgischen Manipulators, um einen chirurgischen Manipulator (4) zu manipulieren, der einen distalendigen Abschnitt (8, 9) für ein Behandlungsinstrument umfasst, der mindestens ein elektrisch manipuliertes Gelenk und einen manuellen Bewegungsmechanismus (10) umfasst, der die Position des distalendigen Abschnitts (8, 9) für ein Behandlungsinstrument bewegt, wobei die Vorrichtung (5) zum Manipulieren eines chirurgischen Manipulators umfasst:
einen manuellen Manipulierabschnitt (16), der den Bewegungsmechanismus (10) manipuliert; und
einen elektrischen Manipulierabschnitt (17), der das Gelenk des distalendigen Abschnitts (8, 9) für ein Behandlungsinstrument manipuliert;
wobei:
der manuelle Manipulierabschnitt (16) einen bewegbaren Abschnitt (16c) umfasst, der den distalendigen Abschnitt (8, 9) für ein Behandlungsinstrument gemäß einem Verschiebungsbetrag bewegt,
der elektrische Manipulierabschnitt (17) einen Manipulationseingabeabschnitt (17b, 17c) umfasst, der das Gelenk antreibt und an dem bewegbaren Abschnitt (16c) befestigt werden kann, wobei
der chirurgische Manipulator (4) einen flexiblen Abschnitt (13) umfasst, in dem der distalendige Abschnitt (8, 9) für ein Behandlungsinstrument an einem distalen Ende desselben befestigt ist,
der bewegbare Abschnitt (16c) an dem flexiblen Abschnitt (13) befestigt ist, um den flexiblen Abschnitt (13) in einer Längsrichtung desselben bewegen zu können, der Bewegungsmechanismus (10) einen Biegeabschnitt (10a, 10b) umfasst, der den distalendigen Abschnitt (8, 9) für ein Behandlungsinstrument schwenkt, der manuelle Manipulierabschnitt (16) einen Basisabschnitt (16a) und einen Schwenkabschnitt (16b) umfasst, der an dem Basisabschnitt (16a) schwenkbar angebracht ist;
der bewegbare Abschnitt (16c) an dem Basisabschnitt (16a) schwenkbar angebracht ist, mit dem Biegeabschnitt (10a, 10b) über ein Kabel (26) verbunden ist und durch den Schwenkabschnitt (16b) getragen wird, um entlang einer Längsrichtung desselben bewegbar zu sein,
die Vorrichtung (5) zum Manipulieren eines chirurgischen Manipulators ferner einen Führungsschlauch (12) umfasst, der das Kabel (26) in einem Zustand aufnimmt, in dem das Kabel (26) durch diesen hindurchgeht, und der an einem distalen Ende desselben mit dem Bewegungsmechanismus (10) verbunden ist, der den Biegeabschnitt (10a, 10b) umfasst, und
ein basales Ende des Führungsschlauchs (12) an dem Basisabschnitt (16) befestigt ist, und ein Ende des Kabels (26) an dem Schwenkabschnitt (16b) befestigt ist.

2. Vorrichtung (5) zum Manipulieren eines chirurgischen Manipulators nach Anspruch 1, wobei der bewegbare Abschnitt (16c) in einer zylindrischen Form gebildet ist, durch die der flexible Abschnitt (13) hindurchgeht.

3. Vorrichtung (5) zum Manipulieren eines chirurgischen Manipulators nach Anspruch 1 oder 2, ferner umfassend:
einen Bewegungseinschränkungsmechanismus (25), der einen Bewegungsbereich des bewegbaren Abschnitts (16c) in einer Längsrichtung im Verhältnis zu dem Schwenkabschnitt (16b) einschränkt.

4. Vorrichtung (5) zum Manipulieren eines chirurgischen Manipulators nach einem der Ansprüche 1 bis 3, ferner umfassend:
einen Blockierungsmechanismus (29), der den Schwenkabschnitt (16b) in einer beliebigen Schwenkposition im Verhältnis zum Basisabschnitt (16a) blockiert.

5. Vorrichtung (5) zum Manipulieren eines chirurgischen Manipulators nach einem der Ansprüche 1 bis 4, wobei der elektrische Manipulierabschnitt (17) an dem bewegbaren Abschnitt (16c) anbringbar/abnehmbar befestigt ist.

6. Vorrichtung (5) zum Manipulieren eines chirurgischen Manipulators nach einem der Ansprüche 1 bis 5, ferner umfassend:
einen Manipuliertisch (15), an dem der Basisabschnitt (16a) angebracht ist, wobei der chirurgische Manipulator (4) eine Motoreinheit (14) umfasst, die das Gelenk an einem basalen Ende des flexiblen Abschnitts (13) antreibt, und
die Motoreinheit (14) an dem Manipuliertisch (15) schwenkbar angebracht ist.

7. Vorrichtung (5) zum Manipulieren eines chirurgischen Manipulators nach Anspruch 6, wobei der Basisabschnitt (16a) an dem Manipuliertisch (15) anbringbar/abnehmbar angebracht ist.

8. Vorrichtung (5) zum Manipulieren eines chirurgischen Manipulators nach Anspruch 1 oder 2,
wobei das basale Ende des Führungsschlauchs (12) an dem Basisabschnitt (16a) anbringbar/abnehmbar befestigt ist, und
die Vorrichtung (5) zum Manipulieren eines chirurgischen Manipulators ferner einen säulenförmigen Abschnitt (27) umfasst, an dem das eine Ende des Kabels (26) befestigt ist und um den herum das Kabel (26) gewickelt ist, wobei der säulenförmige Abschnitt an dem Schwenkabschnitt (16b) anbringbar/abnehmbar angebracht ist.

9. Chirurgisches Manipulatorsystem (1), umfassend:
eine Vorrichtung (5) zum Manipulieren eines chirurgischen Manipulators nach einem der Ansprüche 1 bis 8.

## Revendications

1. Dispositif de manipulation de manipulateur chirurgical (5) pour manipuler un manipulateur chirurgical (4) comprenant une partie d'extrémité distale d'outil de traitement (8, 9) ayant au moins une articulation manipulée électriquement et un mécanisme de déplacement manuel (10) qui déplace la position de la partie d'extrémité distale d'outil de traitement (8, 9), le dispositif de manipulation de manipulateur chirurgical (5) comprenant :
une partie de manipulation manuelle (16) qui manipule le mécanisme de déplacement (10) ; et
une partie de manipulation électrique (17) qui manipule l'articulation de la partie d'extrémité distale d'outil de traitement (8, 9) ;
la partie de manipulation manuelle (16) comprenant une partie mobile (16c) qui déplace la partie d'extrémité distale d'outil de traitement (8, 9) en fonction d'une grandeur de déplacement,
la partie de manipulation électrique (17) comprenant une partie d'entrée de manipulation (17b, 17c) qui entraîne l'articulation, et pouvant être fixée à la partie mobile (16c),
le manipulateur chirurgical (4) comprenant une partie souple (13) dans laquelle la partie d'extrémité distale d'outil de traitement (8, 9) est fixée à une extrémité distale de celle-ci,
la partie mobile (16c) étant fixée à la partie souple (13) de façon à pouvoir déplacer la partie souple (13) dans une direction longitudinale de celle-ci,
le mécanisme de déplacement (10) comprenant une partie de courbure (10a, 10b) qui fait pivoter la partie d'extrémité distale d'outil de traitement (8, 9),
la partie de manipulation manuelle (16) comprenant une partie de base (16a) et une partie de pivotement (16b) qui est attachée à la partie de base (16a) d'une manière pivotante ;
la partie mobile (16c) étant attachée à la partie de base (16a) d'une manière pivotante, étant reliée à la partie de courbure (10a, 10b) par l'intermédiaire d'un fil (26) et étant supportée par la partie de pivotement (16b) de façon à être mobile le long d'une direction longitudinale de celle-ci,
le dispositif de manipulation de manipulateur chirurgical (5) comprenant en outre un tube de guidage (12) qui reçoit le fil (26) dans un état dans lequel le fil (26) passe à travers celui-ci, et qui est relié, au niveau d'une extrémité distale de celui-ci, au mécanisme de déplacement (10) comprenant la partie de courbure (10a, 10b), et
une extrémité de base du tube de guidage (12) étant fixée à la partie de base (16), et une extrémité du fil (26) étant fixée à la partie de pivotement (16b).

2. Dispositif de manipulation de manipulateur chirurgical (5) selon la revendication 1, dans lequel la partie mobile (16c) a une forme cylindrique à travers laquelle la partie souple (13) passe.

3. Dispositif de manipulation de manipulateur chirurgical (5) selon la revendication 1 ou 2, comprenant en outre :
un mécanisme de limitation de mouvement (25) qui limite une plage de mouvement de la partie mobile (16c) dans une direction longitudinale par rapport à la partie de pivotement (16b).

4. Dispositif de manipulation de manipulateur chirurgical (5) selon l'une quelconque des revendications 1 à 3, comprenant en outre :
un mécanisme de verrouillage (29) qui verrouille la partie de pivotement (16b) dans une position de pivotement arbitraire par rapport à la partie de base (16a).

5. Dispositif de manipulation de manipulateur chirurgical (5) selon l'une quelconque des revendications 1 à 4, dans lequel la partie de manipulation électrique (17) est fixée à la partie mobile (16c) d'une manière attachable/détachable.

6. Dispositif de manipulation de manipulateur chirurgical (5) selon l'une quelconque des revendications 1 à 5, comprenant en outre :
une table de manipulation (15) à laquelle la partie de base (16a) est attachée,
le manipulateur chirurgical (4) comprenant une unité de moteur (14) qui entraîne l'articulation au niveau d'une extrémité de base de la partie souple (13), et
l'unité de moteur (14) étant attachée à la table de manipulation (15) d'une manière pivotante.

7. Dispositif de manipulation de manipulateur chirurgical (5) selon la revendication 6, dans lequel la partie de base (16a) est attachée à la table de manipulation (15) d'une manière attachable/détachable.

8. Dispositif de manipulation de manipulateur chirurgical (5) selon la revendication 1 ou 2, dans lequel
l'extrémité de base du tube de guidage (12) est fixée à la partie de base (16a) d'une manière attachable/détachable, et
le dispositif de manipulation de manipulateur chirurgical (5) comprend en outre une partie en colonne (27) à laquelle l'extrémité du fil (26) est fixée, et autour de laquelle le fil (26) est enroulé, la partie en colonne étant attachée à la partie de pivotement (16b) d'une manière attachable/détachable.

9. Système de manipulateur chirurgical (1) comprenant :
un dispositif de manipulation de manipulateur chirurgical (5) selon l'une quelconque des revendications 1 à 8.
